# EUROPEAN PATENT APPLICATION

(11) **EP 4 272 753 A1**
(43) Date of publication of application: **08.11.2023**
(21) Application number: 21914727.9
(22) Date of filing: 31.12.2021
(51) Int. Cl.: A61K 38/17, C07K 14/705, A61K 9/00, A61K 47/26, C07K 14/715, C07K 19/00, A61P 1/00, A61P 19/02, A61P 17/06, A61P 9/10, A61P 27/02

(54) **FORMULATION CONTAINING SOLUBLE GP130 DIMER AND METHOD FOR USING SAME**

(30) Priority: 31.12.2020 CN 202011624158
(71) Applicant: I-Mab Biopharma (Hangzhou) Co., Ltd., Hangzhou, Zhejiang 310018 (CN)
(72) Inventor: LU, Shuyun, Shanghai, 201210 (CN); ZHANG, Zheru, Hangzhou, Zhejiang 310018 (CN); QIAO, Junhua, Hangzhou, Zhejiang 310018 (CN); ZHU, Jing, Hangzhou, Zhejiang 310018 (CN); WANG, Jing, Hangzhou, Zhejiang 310018 (CN)
(74) Representative: J A Kemp LLP
(86) International application number: PCT/CN2021/143870
(87) International publication number: WO 2022/143999

(57) **Abstract**

Provided in the present invention is an aqueous formulation containing a dimer of two single-stranded gp130-Fc fusion proteins, and histidine salt, trehalose and polysorbate 80. The aqueous formulation can be used to treat inflammatory diseases or IL-6-mediated conditions.

## Description

### Technical Field

The present invention belongs to the field of biopharmaceutical research, and specifically relates to a formulation containing a gp130 dimer and the use thereof for the treatment of various IL-6-mediated conditions including an inflammatory disease and cancer.

### Background Art

Glycoprotein 130 (also known as gp130, IL6ST, IL6-beta or CD130) is a transmembrane protein. It forms one subunit of a type I cytokine receptor within the IL-6 receptor family and is very important for signal transduction following cytokine engagement. Structurally, gp130 consists of five fibronectin type-III domains and one immunoglobulin-like C2-type domain in its extracellular portion.

All members of the IL-6 receptor family form complexes with gp130 for signal transduction. For example, IL-6 binds to an IL-6 receptor, and then the complex formed by these two proteins is associated with gp130. After that, the complex consisting of the three proteins is homodimerized to form a hexameric complex which can generate downstream signals.

IL-6 is a pleiotropic cytokine generated by a hematopoietic cell and a non-hematopoietic cell, for example, in response to infections and tissue injuries. IL-6 exerts its multiple biological activities by means of two main signal transduction pathways, including the so-called classic ligand-receptor pathway via a membrane-bound IL-6R mainly existing on hepatocytes and some leukocytes, and the trans-signal transduction pathway (the trans-signaling pathway) via a circulating sIL-6R (soluble IL-6R) derived from proteolytic cleavage of a membrane-bound IL-6R or derived from alternative splicing.

In the classic pathway, the IL-6 directly binds to the membrane-bound IL-6R on the surfaces of a limited range of cell types. The IL-6/IL-6R complex is associated with a dimer preformed by a signal-transducing gp130 receptor protein, which causes a gp130 homodimer to change spatially and thus triggers an intracellular signal transduction cascade. Classic signal transduction is responsible for an acute inflammation defense mechanism and key physiological IL-6 functions, such as the growth and regeneration signals of enterocytes. The extracellular domains of IL-6R and gp130 can be generated by translating an alternatively-spliced mRNA, and no membrane-anchored domains exist, thereby generating sIL-6R and gp130 variants.

The activity of the IL-6/sIL-6R complex is usually controlled by high-level sgpl30 (soluble gp130) existing in circulation, and this complex effectively competes with membrane-bound gp130. The gp130 dimer in the present invention has a higher binding affinity in comparison with the natural sgpl30 and thus has a stronger ability to inhibit IL-6 signal transduction. The formulation of the present invention enables the gp 130 dimer to be more stable during production, transportation and application.

### Summary of the Invention

In order to overcome the problems existing in the prior art, the present invention describes a formulation containing a gp130 dimer (or "fusion protein containing gp130", or "fusion protein" for short), and the use thereof for the treatment of various IL-6-mediated conditions including an inflammatory disease and cancer. The formulation comprises a histidine salt buffer system, has high stability at a pH of about 7.6, and can be safely administered to humans at various doses.

In one embodiment, this description describes an aqueous formulation (also can be referred to as a liquid formulation) and a lyophilized formulation, comprising a fusion protein, a 20-30 mM histidine salt, 220-280 mM trehalose and 0.01(w/v)%-0.03(w/v)% polysorbate 80 and having a pH of 7.0-8.2, wherein the fusion protein comprises two monomers having amino acid sequences as set forth in SEQ ID NO: 1, and the two monomers are connected by a plurality of disulfide bonds. In some embodiments, the aqueous formulation comprises the fusion protein at a concentration of at least 10 mg/mL, 15 mg/mL, 20 mg/mL, 25 mg/mL or at least 30 mg/mL.

In some embodiments, the aqueous formulation has a pH of 7.4-7.8. In some embodiments, the aqueous formulation has a pH of 7.6.

In some embodiments, the aqueous formulation comprises a 24-26 mM histidine salt. In some embodiments, the aqueous formulation comprises a 25 mM histidine salt.

In some embodiments, the aqueous formulation comprises 240-260 mM trehalose. In some embodiments, the aqueous formulation comprises 250 mM trehalose.

In some embodiments, the aqueous formulation comprises 0.015(w/v)%-0.025(w/v)% polysorbate 80. In some embodiments, the aqueous formulation comprises 0.02(w/v)% polysorbate 80.

In some embodiments, the aqueous formulation further comprises a tonicity agent (also known as an osmo-regulator or a stabilizer), a surfactant, an antioxidant, a preservative or a mixture thereof.

In some embodiments, each of the fusion protein molecules comprises no more than six galactose-α-1,3-galactose moieties. In some embodiments, each of the fusion protein molecules comprises no more than three, two or one galactose-α-1,3-galactose moiety.

In some embodiments, the fusion protein comprises glycans, wherein on average at least 52% of the glycans comprise one or more sialic acid residues. In some embodiments, the fusion protein comprises glycans, wherein on average at least 54% of the glycans comprise one or more sialic acid residues. In some embodiments, the fusion protein comprises glycans, wherein on average 52%-65% of the glycans comprise one or more sialic acid residues.

In a preferred embodiment, the aqueous formulation comprises the fusion protein at a concentration of at least 25 mg/mL, a 24-26 mM histidine salt, 240-260 mM trehalose and 0.015(w/v)%-0.025(w/v)% polysorbate 80, with a pH of 7.6-7.8.

In a preferred embodiment, the aqueous formulation comprises the fusion protein at a concentration of 30 mg/mL, a 25 mM histidine salt, 250 mM trehalose and 0.02(w/v)% polysorbate 80, with a pH of 7.6.

In some embodiments, the aqueous formulation comprises no additional amino acid salts at a concentration higher than 10 mM (or higher than 5 mM, 2 mM, 1 mM, 0.1 mM or 0.01 mM) other than the histidine salt, or preferably the aqueous formulation comprises no additional amino acid salts at all.

In some embodiments, the aqueous formulation comprises no additional sugars at a concentration higher than 10 mM (or higher than 5 mM, 2 mM, 1 mM, 0.1 mM or 0.01 mM) other than the trehalose, or preferably the aqueous formulation comprises no additional sugars at all.

In some embodiments, the aqueous formulation is used for the treatment of an inflammatory disease or an IL-6-mediated condition in a human. In some embodiments, the inflammatory disease or IL-6-mediated condition is an inflammatory bowel disease, preferably the treatment induces amelioration of the inflammatory bowel disease. In some embodiments, the inflammatory bowel disease is Crohn's disease or ulcerative colitis, preferably the treatment maintains amelioration of the inflammatory bowel disease. In some embodiments, the inflammatory disease or IL-6-mediated condition is rheumatoid arthritis, psoriasis, uveitis or atherosclerosis. In some embodiments, the inflammatory disease or IL-6-mediated condition is colitis unrelated to inflammatory bowel disease, preferably the colitis is radiation colitis, diverticular colitis, ischemic colitis, infectious colitis, celiac disease, autoimmune colitis or colitis caused by an allergy affecting the colon.

This description also describes a dry formulation, which can be obtained by lyophilizing any of the aqueous formulations described herein or can generate any of the aqueous formulations described herein by adding water.

### Brief Description of the Drawings

FIG. 1 shows DSC results of pH/buffer system screening.
FIG. 2 shows HT-DLS results of pH/buffer system screening.
FIG. 3 shows DSC results of excipient and surfactant screening.
FIG. 4 shows appearances of lyophilized products having different formulas.

### Detailed Description of Embodiments

### Definitions

All numerical designations (e.g., pH, temperature, time, concentration and molecular weight, including ranges) are approximations which are varied (1) or (-) by increments of 0.1% or 10%. It should be understood that, although not always explicitly stated, all numerical designations are preceded by the term "about". It also should be understood that, although not always explicitly stated, the reagent described herein is only exemplary and its equivalents are known in the art.

The terms "protein" and "polypeptide" are used interchangeably and, in their broadest sense, refer to a compound of two or more subunit amino acids, amino acid analogs or peptidomimetics. The subunits may be linked by peptide bonds. In another embodiment, the subunits may be linked by other bonds, e.g., ester and ether. The protein or peptide must comprise at least two amino acids, and no limitation is placed on the maximum number of amino acids that can comprise the protein's or peptide's sequence. As used herein, the term "amino acid" refers to natural and/or unnatural or synthetic amino acids, including glycine and both D and L optical isomers, amino acid analogs and peptidomimetics. Single-letter and three-letter abbreviations of naturally occurring amino acids are listed below.

The "composition" is intended to refer to a combination of an active agent and another inert (e.g., a detectable reagent or label) or active compound or composition (e.g., an adjuvant). The "pharmaceutical composition" is intended to comprise a combination of an active agent with an inert or active carrier, making the composition suitable for diagnostic or therapeutic use in-vitro, in-vivo or ex-vivo.

The "aqueous formulation" refers to a liquid formulation using water as a solvent. In one embodiment, the aqueous formulation is a formulation that does not require lyophilizing, spray-drying and/or freezing to maintain stability (e.g., chemical and/or physical stability and/or biological activity).

As used herein, the term "buffer" represents a pharmaceutically acceptable excipient, which stabilizes the pH of a pharmaceutical formulation. Suitable buffers are well known in the art and can be found in the literature. Pharmaceutically acceptable buffers include, but are not limited to, a tris buffer, an arginine buffer, a histidine buffer, a citrate buffer, a succinate buffer and a phosphate buffer. Independent of the buffer used, the pH can be adjusted with an acid or base known in the art, such as succinic acid, hydrochloric acid, acetic acid, phosphoric acid, sulfuric acid, citric acid, succinate, citrate, tris base, histidine, histidine HCl, sodium hydroxide and potassium hydroxide. Suitable buffers include, but are not limited to, a histidine buffer, 2-morpholinoethanesulfonic acid (MES), dimethyl arsenate, phosphate, acetate, succinate and citrate. The concentration of the buffer may be about 4 mM to about 60 mM, or alternatively about 4 mM to about 40 mM, or alternatively about 5 mM to about 25 mM.

As used herein, the terms "treatment", "treating" and "treat" refer to reversing, alleviating, delaying the onset of or inhibiting the progress of a disease or condition as described herein or one or more symptoms thereof. In some embodiments, treatment may be administered after one or more symptoms are developed. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms are relieved, for example, to prevent or delay their recurrence.

Explanations of proper nouns or terms in each subject matter of the present invention and embodiments are all in common use and are therefore not be reiterated here.

### Formulation

IM001 is a dimer containing two single-stranded gp130-Fc fusion proteins, and can be used for the treatment of various IL-6-mediated conditions including an inflammatory disease and cancer. Similar to other protein drugs, the solubility, stability and activity of IM001 are affected by the environment. Therefore, it would be not easy to develop a suitable formulation comprising a suitable buffer system.

The inventor prepares 12 kinds of pH/buffer system formulas **(Table 2)** and 9 kinds of excipient and surfactant (aqueous formulation) screening formulas **(Table 7),** investigates the stability thereof at 30°C for 2 weeks and compares these formulas by means of DSC, DLS, appearance, protein concentration, pH, SEC-HPLC and SDS (reducing/non-reducing) methods. It has been found that at pH ≤ 6.5, visible particles are obviously generated and the protein is also very unstable, whereas the protein is more stable in a buffer system having a pH of 8.0 (slightly alkaline). In addition, with regard to a same buffer system having a pH of 8.0, the stability in the histidine buffer system is higher than that in the glycine and Tris buffer systems. Interestingly, adding IM001 to a buffer system may lead to pH drift, and after the protein is added to a histidine buffer system, the pH drifts from 7.6 to 8.0.

The inventor of the present application, after investigating various aqueous formulation formulas, finds that the protein at a concentration of 15 mg/mL leads to the best stability under the protection of sucrose and polysorbate 80. Unfortunately, the stability is insufficient when the protein concentration reaches 30 mg/mL.

This result is very unexpected because a stable aqueous formulation having a higher concentration can be easily developed from many proteins comprising Fc fragments, such as an antibody. Without being bound by any particular theory, the inventor of the present application believes that because the fusion protein molecular has a characteristic of being easily unstable at high temperature or high concentration and isoelectric points set limitations on the selections of pH buffer systems, the development of the aqueous formulation is more challenging than the development of biologics molecular formulations such as common monoclonal antibodies.

In order to improve the stability of a high-concentration formulation formula, the inventor of the present application prepares 5 lyophilized formulation screening formulas (Table 14) and investigates the stability thereof at 25°C and 40°C. The experimental results show that the stability of the lyophilized products is obviously improved in comparison with the solution formula at the same (high) concentration of 30 mg/mL. Interestingly, the lyophilized formula (30 mg/mL 25 mM His, 250 mM Trehalose and 0.02% PS80, pH 7.6) shows good results at different temperatures in various tests, and particularly the results are generally not changed at 40°C over the past 2 months. Such results are very unexpected, because generally speaking, the same formulation does not always have advantages in various tests. Therefore, this formula is used for the lyophilized formulation of the IM001. Moreover, the sugar (trehalose) used in this lyophilized formulation formula is also different from the preferred solution (sucrose is used) in the aqueous formulation formula.

Based on these test results, the present application provides an aqueous formulation and lyophilized formulation suitable for IM001, comprising a fusion protein, a histidine salt, trehalose and polysorbate. In some embodiments, the aqueous formulation can form the lyophilized formulation after being lyophilized. In some embodiments, the lyophilized formulation can generate the aqueous formulation by adding an appropriate amount of water. Such an aqueous formulation may also be injected into a patient for the treatment of corresponding diseases.

As mentioned above, the fusion protein (IM001) here comprises two monomers having amino acid sequences as set forth in SEQ ID NO: 1, and the two monomers are connected by a plurality of disulfide bonds. In some embodiments, each of the fusion protein molecules comprises no more than six galactose-α-1,3-galactose moieties. In some embodiments, each of the fusion protein molecules comprises no more than three, two or one galactose-α-1,3-galactose moiety. In some embodiments, the fusion protein comprises glycans, wherein on average at least 52% of the glycans comprise one or more sialic acid residues. In some embodiments, the fusion protein comprises glycans, wherein on average at least 54% of the glycans comprise one or more sialic acid residues. In some embodiments, the fusion protein comprises glycans, wherein on average 52%-65% of the glycans comprise one or more sialic acid residues.

In some embodiments, the aqueous formulation comprises the fusion protein at a concentration of at least 10 mg/mL, 15 mg/mL, 20 mg/mL, 25 mg/mL or at least 30 mg/mL. In some embodiments, the aqueous formulation comprises the fusion protein at a concentration of 10-60 mg/mL, 15-45 mg/mL, 20-40 mg/mL, 25-35 mg/mL or 30 mg/mL.

In some embodiments, the aqueous formulation comprises a histidine salt at a concentration of at least 10 mM. In some embodiments, the aqueous formulation comprises a histidine salt at a concentration of at least 15 mM, 20 mM, 25 mM, 30 mM or 35 mM. In some embodiments, the aqueous formulation comprises a 10-50 mM histidine salt, or a 10-40 mM, 15-35 mM, 20-30 mM, 22-28 mM or 24-26 mM histidine salt. In some embodiments, the aqueous formulation comprises a 25 mM histidine salt.

In some embodiments, the aqueous formulation comprises trehalose at a concentration of at least 100 mM. In some embodiments, the aqueous formulation comprises trehalose at a concentration of at least 100 mM, 150 mM, 200 mM, 250 mM or 300 mM. In some embodiments, the aqueous formulation comprises 100-400 mM trehalose, or 150-350 mM, 200-300 mM, 220-280 mM, 240-260 mM or 245-255 mM trehalose. In some embodiments, the aqueous formulation comprises 250 mM trehalose.

In some embodiments, the aqueous formulation comprises polysorbate, such as polysorbate 20 or polysorbate 80. In some embodiments, the aqueous formulation comprises polysorbate at a concentration of at least 0.005(w/v)%. In some embodiments, the aqueous formulation comprises polysorbate at a concentration of at least 0.01(w/v)%, at least 0.015(w/v)%, at least 0.02(w/v)%, or at least 0.025(w/v)%. In some embodiments, the aqueous formulation comprises 0.01(w/v)%-0.03(w/v)% polysorbate 80. In some embodiments, the aqueous formulation comprises 0.015(w/v)%-0.025(w/v)% polysorbate 80. In some embodiments, the aqueous formulation comprises 0.018(w/v)%-0.022(w/v)% polysorbate 80. In some embodiments, the aqueous formulation comprises 0.019(w/v)%-0.021(w/v)% polysorbate 80. In some embodiments, the aqueous formulation comprises 0.02(w/v)% polysorbate 80.

In some embodiments, the aqueous formulation has a pH of 7.0 or higher. In some embodiments, the aqueous formulation has a pH of 7.1 or higher, 7.2 or higher, 7.3 or higher, 7.4 or higher, 7.5 or higher, or 7.6 or higher. In some embodiments, the aqueous formulation has a pH of 7.0-8.2, or 7.1-8.1, 7.2-8.0, 7.3-7.9, 7.4-7.8 or 7.5-7.7 or 7.55-7.65. In some embodiments, the aqueous formulation has a pH of 7.6.

In an exemplary embodiment, the aqueous formulation comprises the fusion protein at a concentration of at least 25 mg/mL, a 24-26 mM histidine salt, 240-260 mM trehalose and 0.015(w/v)%-0.025(w/v)% polysorbate 80, with a pH of 7.4-7.8. In an exemplary embodiment, the aqueous formulation consists of the fusion protein at a concentration of at least 25 mg/mL, a 24-26 mM histidine salt, 240-260 mM trehalose and 0.015(w/v)%-0.025(w/v)% polysorbate 80, with a pH of 7.4-7.8.

In an exemplary embodiment, the aqueous formulation comprises the fusion protein at a concentration of at least 25 mg/mL, a 25 mM histidine salt, 250 mM trehalose and 0.02(w/v)% polysorbate 80, with a pH of 7.6. In an exemplary embodiment, the aqueous formulation consists of the fusion protein at a concentration of 30 mg/mL, a 25 mM histidine salt, 250 mM trehalose and 0.02(w/v)% polysorbate 80, with a pH of 7.6.

In some embodiments, the aqueous formulation further comprises a tonicity agent (also called an osmo-regulator or a stabilizer), a surfactant, an antioxidant, a preservative or a mixture thereof.

In some embodiments, the aqueous formulation further comprises a tonicity agent (also called an osmo-regulator or a stabilizer). As used herein, the term "tonicity agent" represents a pharmaceutically acceptable agent for regulating the tonicity of the formulation. Isotonicity generally relates to an osmotic pressure relative to a solution, usually relative to a solution of human serum. The formulation may be hypotonic, isotonic or hypertonic. In one aspect, the formulation is isotonic. The isotonic formulation is a liquid, or a liquid reconstituted from a solid form (e.g., from a lyophilized form) and represents a solution which has the same tonicity as some other solutions (e.g., a physiological salt solution and serum) for comparison. Suitable isotonic agents include, but are not limited to, sodium chloride, potassium chloride, glycerol, mannitol and any component from amino acids and sugars as defined herein, and a combination thereof.

In some embodiments, the aqueous formulation further comprises a surfactant. As used herein, the term "surfactant" refers to a pharmaceutically acceptable organic substance having an amphipathic structure, that is, the surfactant consists of groups with opposite solubility trends, generally oil-soluble hydrocarbon chains and water-soluble ionic groups. Surfactants can be classified, depending on the charge of the surface-active moiety, into anionic surfactants, cationic surfactants and nonionic surfactants. Surfactants are usually used as a wetting agent, an emulsifier, a solubilizer and a dispersant for various pharmaceutical compositions and biomaterial formulations. In some embodiments of the pharmaceutical formulation described herein, the amount of the surfactant is described as a percentage (w/v%) expressed in the weight/volume percent. Suitable pharmaceutically acceptable surfactants include, but are not limited to, a group consisting of polyoxyethylene sorbitan fatty acid ester (Tween), polyoxyethylene alkyl ether, alkyl phenyl polyoxyethylene ether (Triton-X), a polyoxyethylene-polyoxypropylene copolymer (poloxamer, Pluronic) or sodium dodecyl sulfate (SDS). The polyoxyethylene sorbitan fatty acid ester comprises polysorbate 20 (sold under the trademark Tween 20^{™}) and polysorbate 80 (sold under the trademark Tween 80^{™}). The polyethylene-polypropylene copolymer comprises those sold under the name Pliironic^{®}F68 or poloxamer 188^{™}. The polyoxyethylene alkyl ether comprises those sold under the trademark Bri j^{™}. The alkyl phenyl polyoxyethylene ether comprises those sold under the trade name Triton-X.

In some embodiments, the aqueous formulation further comprises an antioxidant. The "antioxidant" refers to a molecule capable of slowing down or preventing the oxidation of other molecules. Oxidation is a chemical reaction that transfers electrons from a substance to an oxidant. Oxidation reactions can produce free radicals, which start chain reactions that make a protein therapeutic agent instable and eventually affect the activity of a product. Antioxidants terminate these chain reactions by removing free radical intermediates and inhibits other oxidation reactions by being oxidized themselves. Therefore, antioxidants are usually reducing agents, chelating agents and oxygen scavengers, such as citrate, EDTA, DPTA, mercaptan, ascorbic acid or polyphenol. Nonlimiting examples of antioxidants comprise ascorbic acid (AA, E300), thiosulfate, methionine, tocopherol (E306), propyl gallate (PG, E310), tert-butylhydroquinone (TBHQ), butylated hydroxyanisole (BHA, E320) and butylated hydroxytoluene (BHT, E321).

In some embodiments, the aqueous formulation further comprises a preservative. The "preservative" is a natural or synthetic chemical that is added to products such as foods, pharmaceuticals, paints, biological samples and wood to prevent decomposition resulting from microbial growth or undesirable chemical changes. The preservative additive can be used alone or in combination with other preservation methods. The preservative may be an antimicrobial preservative which inhibits the growth of bacteria and fungi, or an antioxidant such as an oxygen scavenger which inhibits the oxidation of components. Common antimicrobial preservatives comprise benzalkonium chloride, benzoic acid, cholorohexidine, glycerin, benzoic acid, potassium sorbate, thimerosal, sulfite (sulfur dioxide, sodium bisulfite, potassium bisulfite, etc.) and disodium EDTA. Other preservatives comprise those commonly used for parenteral proteins, such as benzyl alcohol, phenol, m-cresol, chlorobutanol or methylparaben.

As can be seen from the embodiments, some commonly used excipients of biologics formulations, such as glycine, Tris and mannitol, do not contribute to the formation of an excellent aqueous formulation or lyophilized formulation. Therefore, in some embodiments, the aqueous formulation does not contain these excipients.

In some embodiments, the aqueous formulation comprises no additional amino acid salts at a concentration higher than 10 mM (or higher than 5 mM, 2 mM, 1 mM, 0.1 mM or 0.01 mM), such as arginine, lysine, asparagine, glutamine, glycine or salts thereof, other than the histidine salt. In some embodiments, the aqueous formulation comprises no additional amino acid salts, such as arginine, lysine, asparagine, glutamine, glycine or salts thereof, other than the histidine salt.

In some embodiments, the aqueous formulation comprises no additional sugars at a concentration higher than 10 mM (or higher than 5 mM, 2 mM, 1 mM, 0.1 mM or 0.01 mM), such as sucrose, other than the trehalose. In some embodiments, the aqueous formulation comprises no additional sugars, such as sucrose, other than the trehalose.

In some embodiments, this description also describes a corresponding dry formulation, such as a lyophilized formulation. In some embodiments, the dry formulation can be obtained by lyophilizing the aqueous formulation described in this description. In some embodiments, the dry formulation can generate the aqueous formulation described in this description by adding an appropriate amount of water.

### Use

The formulation described in this description can be used for the treatment of various corresponding diseases. The gp130 dimer in the present invention has a higher binding affinity in comparison with the natural soluble gpl30 and thus has a stronger ability to inhibit IL-6 signal transduction. IL-6 signal transduction is related to many diseases, comprising diseases briefly described below and generally understood by those skilled in the art.

Chronic inflammation, such as Crohn's disease (CD), ulcerative colitis (UC), rheumatoid arthritis (RA) or psoriasis is histologically related to the presence of mononuclear cells such as macrophages and lymphocytes, which persist in tissues after having been acquired for the resolution of the acute inflammatory phase. In models of chronic inflammatory diseases, IL-6 seems to have detrimental role favoring mononuclear cell accumulation at the site of injury by inducing sustained MCP-1 secretion, angioproliferation and antiapoptotic functions on T cells.

Inflammatory bowel disease (IBD), namely CD or UC, is a chronic inflammation occurring in the intestinal tract of a susceptible individual, and is believed to be unrelated to a specific pathogen. Alterations in the epithelial mucosal barrier, accompanied by the increase in intestinal permeability, lead to enhanced exposure of the mucosal immune system to intestinal lumen antigens, which results in inappropriate activation of the intestinal immune systems in patients. The uncontrolled activation of mucosal CD4+T-lymphocytes, accompanied by continuous excessive release of proinflammatory cytokines, induces pathogenic gastrointestinal inflammations and tissue injuries. There is a consensus that the main activated immune cells involved in the pathogenesis of IBD are intestinal T cells and macrophages.

IL-6 is shown as a central cytokine in IBD in the human body. It has been found that (3) and UC patients generate increased levels of IL-6 in comparison with the control group, and the level of the IL-6 is related to clinical activities. It has also been found that the level of SIL-6R in (3) patients is increased, and accordingly, the level of the IL-6/sIL-6R complex in serum is increased. Lamina propria mononuclear cells obtained from surgical colon specimens from CD and UC patients show that both CD4+T cells and macrophages generate increased amounts of IL-6 in comparison with the control group. It has been found that SIL-6R is released via shedding from the surfaces of macrophages and mononuclear cells, which is accompanied by increased production related to the increased level of IL-6. In CD patients, mucosal T cells show strong evidence for IL-6 trans-signal transduction, which is accompanied by activation of STAT3, bcl-2 and bcl-xl. The blockade of the IL-6 trans-signal transduction causes T cell apoptosis, which indicates that the IL-6/sIL-6R system mediates the resistance of T cells to apoptosis in CD.

Therefore, in IBD patients, acquired accumulation of proinflammatory CD4 + T cells, which leads to perpetuation of inflammation, in the lamina propria is critically dependent on anti-apoptotic IL-6/sIL-6R trans-signal transduction. It is believed that the polypeptide disclosed herein can be used for the treatment of CD and other inflammatory diseases by acting on the IL-6/sIL-6R complex.

Therefore, the formulation of the present invention can be used to treat an IL-6-mediated condition. The IL-6-mediated condition comprises an inflammatory disease or cancer. In this regard, the polypeptide and composition described herein may be administered to a subject with an inflammatory disease, such as juvenile idiopathic arthritis, Crohn's disease, colitis (e.g., colitis unrelated to IBD, including radiation colitis, diverticular colitis, ischemic colitis, infectious colitis, celiac disease, autoimmune colitis or colitis resulting from an allergy affecting the colon), dermatitis, psoriasis, uveitis, diverticulitis, hepatitis, irritable bowel syndrome (IBS), lupus erythematosus, nephritis, Parkinson's disease, ulcerative colitis, multiple sclerosis (MS), Alzheimer's disease, arthritis, rheumatoid arthritis, asthma and various cardiovascular diseases such as atherosclerosis and vasculitis. In certain embodiments, the inflammatory disease is selected from a group consisting of diabetes mellitus, gout, cryopyrin-associated periodic syndromes and chronic obstructive pulmonary disease.

Preferably, the inflammatory disease or IL-6-mediated condition is an inflammatory bowel disease, preferably wherein the treatment induces amelioration of the inflammatory bowel disease. Preferably, the inflammatory bowel disease is Crohn's disease or ulcerative colitis, preferably wherein the treatment maintains amelioration of the inflammatory bowel disease. Preferably, the inflammatory disease or IL-6-mediated condition is rheumatoid arthritis, psoriasis, uveitis or atherosclerosis. Preferably, the inflammatory disease or IL-6-mediated condition is colitis unrelated to inflammatory bowel disease, preferably wherein the colitis is radiation colitis, diverticular colitis, ischemic colitis, infectious colitis, celiac disease, autoimmune colitis or colitis resulting from an allergy affecting the colon. Preferably, the inflammatory disease or IL-6-mediated condition is selected from Crohn's disease, ulcerative colitis, rheumatoid arthritis and psoriasis, more preferably from Crohn's disease and ulcerative colitis.

With regard to the inflammatory disease such as an inflammatory bowel disease, the treatment can comprise amelioration of the condition, maintenance of the amelioration of the condition, or both.

Other embodiments provide a method for treating, reducing the severity of or preventing cancers which include, but are not limited to, multiple myeloma, plasma cell leukemia, renal cell carcinoma, Kaposi's sarcoma, colorectal cancer, gastric cancer, melanoma, leukemia, lymphoma, glioma, glioblastoma multiforme, lung cancer (including, but not limited to non-small cell lung cancer (NSCLC; adenocarcinoma and squamous cell carcinoma)), non-Hodgkin's lymphoma, Hodgkin's disease, plasmacytoma, sarcoma, thymoma, breast cancer, prostate cancer, hepatocellular carcinoma, bladder cancer, uterine cancer, pancreatic cancer, esophageal cancer, brain cancer, head and neck cancers, ovarian cancer, cervical cancer, testicular cancer, stomach cancer, esophageal cancer, liver cancer, acute lymphoblastic leukemia (ALL), T-ALL, acute myeloid leukemia (AML), chronic myeloid leukemia (CML), chronic lymphocytic leukemia (CLL), salivary gland cancer or other cancers.

Other embodiments of the present disclosure provide a method for treating, reducing the severity of or preventing a disease which is selected from a group consisting of sepsis, bone resorption (osteoporosis), cachexia, cancer-related fatigue, psoriasis, systemic juvenile idiopathic arthritis, systemic lupus erythematosus (SLE), mesangial proliferative glomerulonephritis, hypergammaglobulinemia, Castleman's disease, IgM gammopathy, cardiac myxoma and autoimmune insulin-dependent diabetes mellitus.

### Production method

In a further aspect, the present disclosure provides a way for producing the formulation. The cDNA encoding SEQ ID NO: 1 may be cloned into a vector such that the signal peptide is linked in-frame to an amino terminus of the amino acid sequence of an antibody chain. The signal peptide may be an immunoglobulin signal peptide or a heterologous signal peptide (i.e., a signal peptide from a non-immunoglobulin protein).

The design of the expression vector, comprising the selection of regulatory sequences, may depend on factors such as the selection of host cells to be transformed and the expression level of desired proteins. Regulatory sequences for the expression of mammalian host cells comprises viral elements that direct high levels of protein expression in mammalian cells, such as promoters and/or enhancers derived from retroviral LTRs, cytomegalovirus (CMV) (such as a CMV promoter/enhancer), simian virus 40 (SV40) (such as an SV40 promoter/enhancer), adenovirus (such as an adenovirus major late promoter (AdMLP)), polyoma and strong mammalian promoters (such as natural immunoglobulin and actin promoters). The host cell may be a mammalian cell, an insect cell, a plant cell, a bacterial cell or a yeast cell, preferably the cell is a mammalian cell, such as a Chinese hamster ovary (CHO) cell. Exemplary CHO cells are (CHO)/dhfr⁻ cells obtained from the European Collection of Authenticated Cell Cultures (ECACC, No. 9406067). Preferably, the host cell is a CHO cell, and the nucleic acid encoding the polypeptide is codon-optimized for use in the CHO cell.

In another aspect, the present disclosure comprises a fusion protein produced by the method disclosed herein. Preferably, the dimer has the characteristics described herein (e.g., % galactose-α-1,3-galactose moieties per mole of polypeptide, sialylation). The dimer generated by the method can be used for the preparation of a suitable composition. The fusion protein molecule produced in this way comprises no more than six galactose-α-1,3-galactose moieties, or no more than three, two or one galactose-α-1,3-galactose moiety.

The fusion protein molecule produced in this way comprises glycans, wherein on average at least 52% of the glycans comprise one or more sialic acid residues. In some embodiments, the fusion protein comprises glycans, wherein on average at least 54% of the glycans comprise one or more sialic acid residues. In some embodiments, the fusion protein comprises glycans, wherein on average 52%-65% of the glycans comprise one or more sialic acid residues.

### Example 1

The IM001 was obtained from a CHO-K1 engineered cell expressing a gp130-Fc fusion protein gene by means of cell culture, separation and purification.

**Table 1. Amino acid sequence of a single strand of IM001**

| Single strand of IM001 (SEQ ID NO: 1) |
|---|
| ELLDPCGYISPESPVVQLHSNFTAVCVLKEKCMDYFHVNANYIVWKTNHFTIPKEQYTIINRTASSVTFTDIASLNI QLTCNILTFGQLEQNVYGITIISGLPPEKPKNLSCIVNEGKKMRCEWDGGRETHLETNFTLKSEWATHKFADCKAKR DTPTSCTVDYSTVYFVNIEVWVEAENALGKVTSDHINFDPVYKVKPNPPHNLSVINSEELSSILKLTWTNPSIKSVI ILKYNIQYRTKDASTWSQIPPEDTASTRSSFTVQDLKPFTEYVFRIRCMKEDGKGYWSDWSEEASGITYEDRPSKAP SFWYKIDPSHTQGYRTVQLVWKTLPPFEANGKILDYEVTLTRWKSHLQNYTVNATKLTVNLTNDRYLATLTVRNLVG KSDAAVLTIPACDFQATHPVMDLKAFPKDNMLWVEWTTPRESVKKYILEWCVLSDKAPCITDWQQEDGTVHRTYLRG NLAESKCYLITVTPVYADGPGSPESIKAYLKQAPPSKGPTVRTKKVGKNEAVLEWDQLPVDVQNGFIRNYTIFYRTI IGNETAVNVDSSHTEYTLSSLTSDTLYMVRMAAYTDEGGKDGPEFTFTTPKFAQGEDKTHTCPPCPAPEAEGAPSVF LFPPKPKDTLMISRTPEVTCVVVDVSHEDPEVKFNWYVDGVEVHNAKTKPREEQYNSTYRVVSVLTVLHQDWLNGKE YKCKVSNKALPAPIEKTISKAKGQPREPQVYTLPPSREEMTKNQVSLTCLVKGFYPSDIAVEWESNGQPENNYKTTP PVLDSDGSFFLYSKLTVDKSRWQQGNVFSCSVMHEALHNHYTQKSLSLSPGK |

The cDNA sequence of the IM001 was expressed in a CHO cell expression system. The presence of the IgGl Cys-Pro-Pro-Cys sequence in the Fc region resulted in the dimerization of two identical gpl30-Fc subunits by means of sulfhydryl residues on the Fc region, which together form the IM001.

The pilot manufacturing and purification process of the IM001 drug substance was as follows. Before inoculated into a bioreactor for production, cells from a WCB vial were revived and gradually expanded using a protein-free culture medium. Upon completion of the cell culture, cells and cell debris were removed by filtration of the culture. Purification consisted of three column chromatography steps, one concentration and diafiltration step and two specific viral clearance steps (viral inactivation treatment and nanofiltration, and removal of enveloped and non-enveloped viruses). Following concentration and diafiltration, an excipient was added to formulate a drug substance. The prepared IM001 was filtered into a container by means of a 0.22-µM filter membrane.

This example also attempted to screen the most stable pH/buffer system for the IM001.

The IM001 stock solutions were exchanged into 20 mM acetate (pH 4.5, pH 5.0 and pH 5.5), citrate (pH 5.0), histidine-aspartate (pH 5.0 and pH 5.5), histidine (pH 5.5, pH 6.0, pH 6.5, pH 7.0 and pH 8.0) and phosphate (pH 7.0) by means of dialysis. The concentration of the protein was adjusted to about 30 mg/mL, and then the protein was filtered with a 0.22-µm PVDF membrane filter. Following filtration, the samples were bottled and sealed. All manipulations took place in a biological safety shield. The sample in one of the bottles was used for T0, and appropriate stability investigation conditions were selected based on the DSC and HT-DLS results of T0. The rest samples were stored under the conditions, and each bottle was subjected to sampling analysis at time points specified in Table 2.

**Table 2. Study protocol for IM001 pH/buffer system screening**

| **Formula no.** | **Buffer system** | **pH of buffer** | **T0** | **30** | |
|---|---|---|---|---|---|
| | | | | **1W** | **2W** |
| 1 | 20 mM acetate buffer system | 4.5 | x,y | y | y |
| 2 | | 5.0 | | | |
| 3 | | 5.5 | | | |
| 4 | 20 mM citrate buffer system | 5.5 | | | |
| 5 | 20 mM histidine-aspartate buffer system | 5.0 | | | |
| 6 | | 5.5 | | | |
| 7 | 20 mM histidine salt buffer system | 5.5 | | | |
| 8 | | 6.0 | | | |
| 9 | | 6.5 | | | |
| 10 | | 7.0 | | | |
| 11 | | 8.0* | | | |
| 12 | 20 mM phosphate buffer system | 7.0 | | | |
| *Buffers had a pH of 8.0, which was changed to 7.6 after the protein was added for medium exchange; | | | | | |
| x = DSC, HT-DLS; | | | | | |
| y = Appearance, protein concentration, pH, SEC-HPLC, SDS-PAGE (reducing/non-reducing). | | | | | |

### 1.1. DSC and HT-DLS results

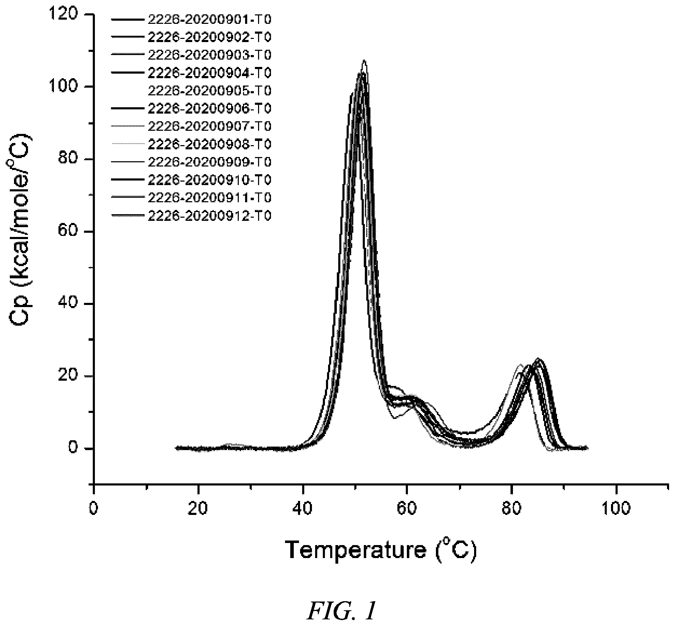

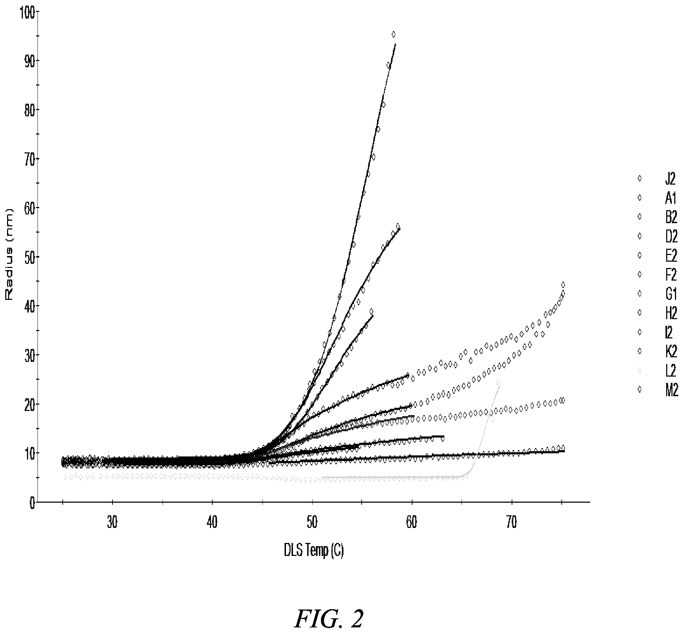

The results were detailed in Table 3 and FIGs. 1-2. The Tm-onset for all formulas was 40.9°C-43.0°C, and except that the Tagg Onset of formula 1 was 60.6°C and the Tagg Onset of formula 11 was 65.7°C, the Tagg Onset for other formulas was about 45°C.

**Table 3. DSC and HT-DLS results of IM001 pH/buffer system screening**

| **NO.** | **DSC result (°C)** | | | | **HT-DLS result** | |
|---|---|---|---|---|---|---|
| | **Tm-onset** | **Tm1** | **Tm2** | **Tm3** | **Tagg onset (°C)** | **Radius (nm)** |
| 1 | 42.0 | 49.4 | 57.4 | 81.1 | 60.6 | 9.1 |
| 2 | 41.8 | 50.2 | 60.4 | 83.1 | 45.3 | 9.7 |
| 3 | 41.8 | 50.7 | 61.4 | 84.2 | 45.0 | 10.1 |
| 4 | 41.8 | 50.7 | 60.9 | 83.4 | 44.7 | 10.6 |
| 5 | 41.3 | 50.2 | 59.9 | 81.4 | 48.1 | 9.5 |
| 6 | 42.2 | 50.6 | 60.7 | 82.7 | 45.5 | 9.9 |
| 7 | 40.9 | 50.3 | 60.0 | 81.2 | 44.6 | 10.0 |
| 8 | 41.9 | 50.8 | 61.4 | 83.5 | 44.4 | 10.1 |
| 9 | 42.4 | 51.2 | 61.7 | 84.5 | 44.2 | 9.9 |
| 10 | 42.8 | 51.3 | 61.9 | 84.8 | 46.0 | 9.2 |
| 11 | 42.7 | 51.5 | 62.1 | 84.6 | 65.7 | 6.0 |
| 12 | 43.0 | 51.5 | 63.4 | 83.6 | 48.8 | 10.1 |

### 1.2. Appearance, pH and protein concentration results

The results were detailed in Table 4. The appearance results showed that all formulas, except for formula 1 and formula 11, had particles observed at T0, and all formulas had particles observed after being stored at 30°C for 1 week and 2 weeks. The pH and protein concentration results showed that all formulas showed no obvious changes after being stored at 30°C for 2 weeks.

**Table 4. Appearance, pH and protein concentration results of IM001 pH/buffer system screening**

| **NO.** | **Appearance** | | | **pH** | | | **Protein concentration (mg/mL)** | | |
|---|---|---|---|---|---|---|---|---|---|
| | **T0** | **30C-1W** | **30C-2W** | **T0** | **30C-1W** | **30C-2W** | **T0** | **30C-1W** | **30C-2W** |
| 1 | SY, SO, FP | SY, SO, PO+ | SY, SO, PO+ | 4.7 | 4.7 | 4.7 | 30.0 | 30.2 | 30.1 |
| 2 | SY, SO, PO+ | SY, SO, PO+ | SY, SO, PO+ | 5.1 | 5.1 | 5.1 | 29.6 | 30.0 | 29.9 |
| 3 | SY, SO, PO+ | SY, SO, PO+ | SY, SO, PO++ | 5.5 | 5.5 | 5.5 | 29.9 | 30.2 | 30.2 |
| 4 | SY, SO, PO+ | SY, SO, PO++ | SY, OL, PO++ | 5.5 | 5.5 | 5.5 | 29.9 | 30.5 | 30.5 |
| 5 | SY, SO, PO+ | SY, SO, PO+ | SY, SO, PO++ | 5.2 | 5.2 | 5.2 | 29.9 | 29.9 | 29.8 |
| 6 | SY, SO, PO+ | SY, SO, PO+ | SY, SO, PO++ | 5.6 | 5.6 | 5.6 | 29.8 | 30.1 | 30.1 |
| 7 | SY, SO, PO++ | SY, SO, PO++ | SY, SO, PO++ | 5.5 | 5.5 | 5.5 | 30.1 | 30.3 | 30.3 |
| 8 | SY, SO, PO++ | SY, SO, PO++ | SY, SO, PO++ | 6.0 | 6.0 | 6.0 | 29.7 | 29.9 | 29.8 |
| 9 | SY, SO, PO+ | SY, SO, PO++ | SY, SO, PO++ | 6.5 | 6.5 | 6.5 | 29.7 | 29.9 | 29.6 |
| 10 | SY, SO, PO+ | SY, SO, PO++ | SY, SO, PO++ | 6.9 | 6.9 | 6.9 | 29.6 | 30.0 | 29.7 |
| 11 | SY, SO, FP | SY, SO, PO+ | SY, SO, PO+ | 7.5 | 7.6 | 7.6 | 29.5 | 29.8 | 29.5 |
| 12 | SY, SO, PO+ | SY, SO, PO+ | SY, SO, PO+ | 7.0 | 7.0 | 7.0 | 30.1 | 30.2 | 30.0 |

| | | | | | | | | | |
|---|---|---|---|---|---|---|---|---|---|
| Notes: SY (slightly yellow)/SO (slightly opalescent liquid)/OL (opalescent liquid), FP (free of visible particles)/PO (particles observed)/+ (a few visible particles)/++ (many visible particles) | | | | | | | | | |

### 1.3. SEC purity results

The results were detailed in Table 5. The results showed that there were different degrees of decrease in the SEC purities of all formulas after storage at 30°C for 1-2 weeks, wherein the degree of decrease of formula 11 was the lowest (about 13%), and the decrease ranges of other buffer systems were 19.3%-72%.

**Table 5. SEC purity results of IM001 pH/buffer system screening**

| **NO.** | **Monomer peak** | | | **Aggregate peak** | | | **Fragment peak** | | |
|---|---|---|---|---|---|---|---|---|---|
| | **T0** | **30C-1W** | **30C-2W** | **T0** | **30C-1W** | **30C-2W** | **T0** | **30C-1W** | **30C-2W** |
| 1 | 90.4 | 36.4 | 18.4 | 2.6 | 18.6 | 20.8 | 7.0 | 45.0 | 60.8 |
| 2 | 92.1 | 48.9 | 32.4 | 2.7 | 17.7 | 20.1 | 5.1 | 33.4 | 47.6 |
| 3 | 93.7 | 59.3 | 43.9 | 2.9 | 15.4 | 18.7 | 3.4 | 25.3 | 37.4 |
| 4 | 92.0 | 48.3 | 34.8 | 2.9 | 19.3 | 19.3 | 5.1 | 32.4 | 45.9 |
| 5 | 93.6 | 57.6 | 42.3 | 2.7 | 14.4 | 16.1 | 3.7 | 28.0 | 41.7 |
| 6 | 94.2 | 65.4 | 52.7 | 3.0 | 14.2 | 16.9 | 2.8 | 20.3 | 30.4 |
| 7 | 93.5 | 59.0 | 44.0 | 3.1 | 15.8 | 17.3 | 3.4 | 25.2 | 38.8 |
| 8 | 95.6 | 69.2 | 58.5 | 3.2 | 14.6 | 17.7 | 1.2 | 16.1 | 23.8 |
| 9 | 95.5 | 74.6 | 66.0 | 3.5 | 15.1 | 19.6 | 1.0 | 10.2 | 14.4 |
| 10 | 95.5 | 82.7 | 76.2 | 3.5 | 14.1 | 18.6 | 0.9 | 3.2 | 5.2 |
| 11 | 95.3 | 85.1 | 82.3 | 3.9 | 12.9 | 15.1 | 0.8 | 2.0 | 2.7 |
| 12 | 94.9 | 80.2 | 73.4 | 4.1 | 17.3 | 21.8 | 1.0 | 2.5 | 4.8 |

### 1.4. SDS-PAGE (reducing/non-reducing) results

The results were detailed in Table 6. The reducing SDS-PAGE results showed that after storage at 30°C for 2 weeks, there was no decrease in purities of p10 (His at pH 7.0), p11 (His at pH 8.0) and p12 (Phosphate at pH 7.0), while there showed different degrees of decrease in purities of the rest samples, with decrease ranges of 10.8%-50.0%. The non-reducing SDS-PAGE results showed that there were different degrees of decrease in purities of all samples, wherein the decrease ranges of p10 (His at pH 7.0), p11 (His at pH 8.0) and p12 (Phosphate at pH 7.0) were less than those of the rest samples which were 14.6%-65.7%.

**Table 6. SDS-PAGE (reducing/non-reducing) results of IM001 pH/buffer system screening**

| **NO.** | **SDS-PAGE (R)** | | | | | | **SDS-PAGE (NR)** | | | | | |
|---|---|---|---|---|---|---|---|---|---|---|---|---|
| | **Purity (%)** | | | **Main Band (kDa)** | | | **Purity (%) (H** + **L)** | | | **Main Band (kDa)** | | |
| | **T0** | **30C-1W** | **30C-2W** | **T0** | **30C-1W** | **30C-2W** | **T0** | **30C-1W** | **30C-2W** | **T0** | **30C-1W** | **30C-2W** |
| 1 | 98.1 | 67.5 | 48.1 | 109 | 104 | 107 | 91.3 | 47.8 | 25.6 | 316 | 262 | 265 |
| 2 | 98.4 | 79.9 | 64.5 | 106 | 102 | 107 | 92.7 | 59.6 | 41.8 | 297 | 252 | 255 |
| 3 | 99.3 | 85.7 | 75.4 | 106 | 100 | 107 | 94.2 | 69.6 | 52.9 | 301 | 246 | 245 |
| 4 | 98.6 | 79.2 | 68.1 | 106 | 100 | 108 | 93.4 | 61.4 | 42.6 | 294 | 252 | 240 |
| 5 | 99.3 | 83.6 | 73.8 | 105 | 98 | 109 | 95.4 | 66.7 | 49.1 | 297 | 262 | 236 |
| 6 | 99.3 | 89.2 | 81.6 | 105 | 98 | 110 | 96.8 | 75.7 | 61.8 | 294 | 272 | 236 |
| 7 | 99.5 | 85.4 | 67.9 | 109 | 107 | 107 | 94.7 | 69.0 | 53.5 | 262 | 216 | 245 |
| 8 | 99.5 | 93.0 | 79.7 | 108 | 105 | 105 | 95.4 | 79.2 | 71.6 | 259 | 212 | 233 |
| 9 | 99.4 | 97.5 | 88.6 | 108 | 105 | 104 | 94.6 | 85.7 | 80.0 | 251 | 205 | 227 |
| 10 | 99.5 | 100.0 | 100.0 | 108 | 107 | 102 | 94.4 | 92.1 | 85.1 | 243 | 205 | 222 |
| 11 | 99.2 | 100.0 | 100.0 | 109 | 108 | 101 | 94.8 | 92.2 | 85.5 | 240 | 210 | 229 |
| 12 | 99.0 | 100.0 | 100.0 | 109 | 110 | 101 | 94.2 | 91.7 | 85.4 | 248 | 228 | 224 |

In this study, the most stable pH/buffer system for the IM001 was screened by means of DSC, DLS, appearance, protein concentration, pH, SEC-HPLC and SDS-PAGE (reducing/non-reducing). The DSC and DLS results showed that the Tm Onset and Tagg Onset of the IM001 molecule showed an increasing trend with the increase of pH, wherein the Tm Onset and Tagg Onset of p11 (His at pH 8.0) were higher than those of the rest samples. After the samples were placed at 30°C for 2 weeks, the appearance, SEC-HPLC and SDS-PAGE (reducing/non-reducing) results showed that the IM001 was relatively unstable in a low pH environment, and formula 11 (the histidine buffer system with a pH of 8.0, which was changed to 7.6 after the protein was contained) was relatively superior to other formulas in each test.

### Example 2

The study purpose of this example was to screen an appropriate excipient stabilizer on the basis of pH/buffer system screening.

Based on the results of the pH/buffer system screening experiments, a 25 mM histidine buffer system at pH 8.0 was used as a main buffer system for the assessment of excipient screening. In addition, the glycine buffer system at pH 7.5 and the Tris buffer system at pH 7.5 were added for investigation. The IM001 stock solution was exchanged into a 25 mM histidine buffer system (pH 8.0), a 25 mM glycine buffer system (pH 7.5) and a 25 mM Tris buffer system (pH 7.5) by means of ultrafiltration centrifugation. Different excipient mother solutions and surfactant mother solutions were added respectively according to the experimental scheme; the protein contents were adjusted to about 30 mg/mL and about 15 mg/mL respectively; and 9 formulas comprising different excipients and surfactants were finally prepared. The samples were filtered respectively with a 0.22-µm PVDF membrane in a biological safety cabinet and then subpackaged into penicillin bottles, and the bottles were plugged and sealed. The experimental investigations under different conditions were started. See Table 7 for specific schemes. The pH of formulas in the table was the pH of buffers without adding protein. Since the pH drifted after the IM001 was added in the buffer system, determination results represented the actual pH of the formulas.

**Table 7. Excipient and surfactant screening schemes**

| **Formula no.** | **Formula** | **Type of stability** | **T0** | **Acceleration** | | | **Long term** | | | | **Freezing and thawing** | **Influence factor** | | **Vibration and shaking** |
|---|---|---|---|---|---|---|---|---|---|---|---|---|---|---|
| | | **Preservation condition** | | **25°C ± 2°C** | | | **2°C to 8°C** | | | | **-70°C to RT** | **30°C ± 2°C** | | **300 rpm to 25°C** |
| | | **Sampling point** | | **2W** | **1M** | **3M** | **1M** | **3M** | **6M** | **12M** | **5C** | **1W** | **2W** | **7D** |
| F1 | 30 mg/mL 25 mM His pH 8.0, 250 mM Sucrose, 0.01% PS20 | | | Y | Y, z | Y, Z | Y | Y, Z | Y, Z | Y, Z | Y. Z | Y | Y. Z | Y, Z |
| F2 | 30 mg/mL 25 mM His pH 8.0, 250 mM Sucrose, 0.02% PS80 | | | Y | Y, Z | Y, Z | Y | Y, Z | Y, G | Y; Z | Y. Z | Y | Y, Z | Y, Z |
| F3 | 30 mg/mL 25 mM His pH 8.0, 250 mM Trehalose. 0.02% PS80 | | | Y | Y, Z | Y, Z | Y | Y, Z | Y, Z | Y, Z | Y, Z | Y | Y, Z | Y, z |
| F4 | 30 mg/mL 25 mM Glycine pH 7.5, 250 mM Trehalose, 0.02% PS80 | | | Y | Y, Z | Y, z | Y | Y, Z | Y, Z | Y, Z | Y, Z | Y | Y, Z | Y, Z |
| F5 | 30 mg/mL 25 mM Tris pH 7.5, 250 mM Trehalose, 0.02% PS80 | Test items | X, Y, Z | Y | Y, Z | Y, Z | Y | Y, Z | Y, Z | Y, Z | Y, Z | Y | Y, Z | Y, Z |
| F6 | 30 mg/mL 25 mM His pH 8.0, 250 mM Trehalose, 0.02% PS80, 1 mM Met | | | Y | Y, Z | Y, Z | Y | Y. Z | Y, Z | Y, Z | Y, Z | Y | Y. Z | Y, Z |
| F7 | 30 mg/mL 25 mM His pH 8.0, 4% mannitol, 2% Sucrose, 0.02% PS80 | | | Y | Y, Z | Y, Z | Y | Y, Z | Y, Z | Y, Z | Y, Z | Y | Y, Z | Y, Z |
| F8 | 30 mg/mL 25 mM His pH 8.0, 4.5% mannitol, 0.02% PS80 | | | Y | Y, Z | Y, Z | Y | Y, Z | Y, Z | Y, Z | Y, Z | Y | Y, Z | Y, Z |
| F9 | 15 mg/mL 25 mM His pH 8.0, 250 mM Sucrose, 0.02% PS80 | | | Y | Y, Z | Y, Z | Y | Y, Z | Y, Z | Y, z | Y, Z | Y | Y, Z | Y, Z |

| | | | | | | | | | | | | | | |
|---|---|---|---|---|---|---|---|---|---|---|---|---|---|---|
| Y = appearance, pH, protein concentration, SEC-HPLC; Z = MFI; X = DSC, osmotic pressure Notes: 1. After the excipient screening samples were investigated at 2°C to 8°C for 1 month, particles were obviously observed in all the formulas except for F5. The purities of raw materials used in this round of excipient screen ing at T0 were low and had limited representativeness, which rendered that sampling and testing were no longer performed at the sampling point after 1 month of investigation. | | | | | | | | | | | | | | |

### 2.1 DSC results of excipient and surfactant screening experiments

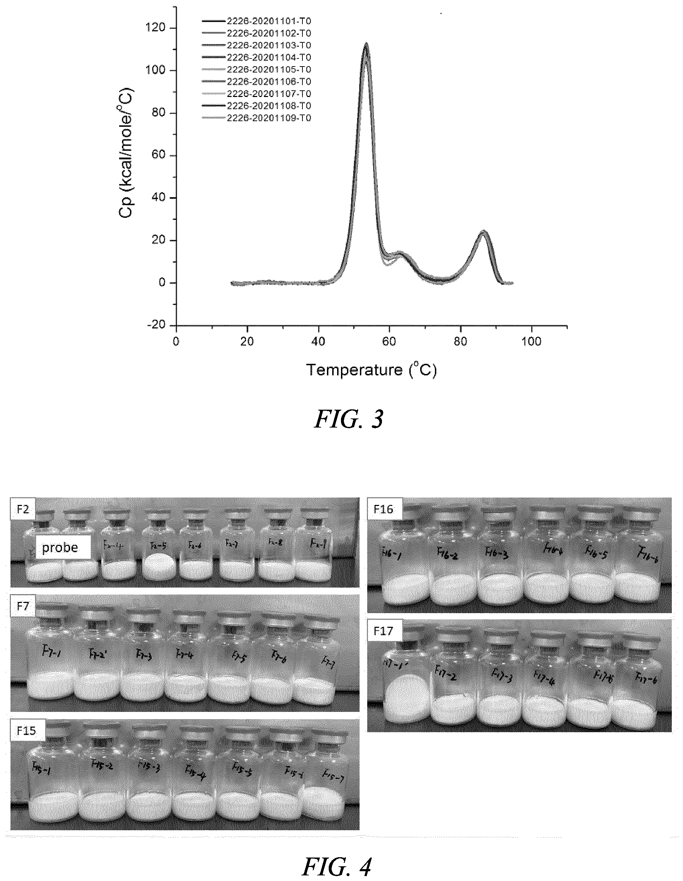

Samples of all formulas were prepared and then tested by means of a DSC. The test results were shown in Table 8, and the DSC curve graph was shown in FIG. 3. The data showed that the Tm Onset of the 9 samples was about 45°C, and the Tm Onset of the samples with different formulas had no significant difference.

**Table 8. DSC results of excipient and surfactant screening experiments**

| **Formula no.** | **Sample no.** | **DSC** | |
|---|---|---|---|
| | | **Tm Onset (°C)** | **Tm1 (°C)** |
| F1 | 2226-20201101 | 45.0 | 53.2 |
| F2 | 2226-20201 102 | 45.5 | 53.3 |
| F3 | 2226-20201103 | 45.0 | 53.4 |
| F4 | 2226-20201104 | 45.5 | 53.4 |
| F5 | 2226-20201105 | 45.4 | 53.2 |
| F6 | 2226-20201106 | 44.8 | 53.3 |
| F7 | 2226-20201107 | 45.1 | 53.1 |
| F8 | 2226-20201108 | 44.1 | 52.9 |
| F9 | 2226-20201109 | 44.0 | 53.3 |

### 2.2 Appearance test results of excipient and surfactant screening experiments

The appearance results of the excipient and surfactant screening experiments were summarized in **Table 9.** The data showed that no visible particles were observed in the 9 formula samples after freezing and thawing for 5 cycles; however, many visible particles appeared in the 9 formula samples under vibration and shaking at 25°C and 300 rpm for 7 days; visible particles appeared in F1 and F4 after placing at high temperature (30°C ± 2°C) for 2 weeks; visible particles appeared in F1, F4 and F5 after placing under acceleration (25°C ± 2°C) conditions for 1 month; and visible particles appeared in all the formulas except for F5 after placing under long-term (2°C to 8°C) conditions for 1 month.

**Table 9 Appearance results of excipient and surfactant screening experiments**

| **Formula no.** | **Sample no.** | **Appearance** | | | | | | | |
|---|---|---|---|---|---|---|---|---|---|
| | | **T0** | **FT-5C** | **A300-7D** | **30-1W** | **30-2W** | **25-2W** | **25-1M** | **05-1M** |
| F1 | 2226-20201101 | SY, SO, FP | SY, SO, FP | SY, SO, PO++ | SY, SO, FP | SY, SO, PO+ | SY, SO, PO++ | SY, SO, PO++ | SY, SO, PO++ |
| F2 | 2226-20201102 | SY, SO, FP | SY, SO, FP | SY, SO, PO++ | SY, SO, FP | SY, SO, FP | SY, SO, FP | SY, SO, FP | SY, SO, PO++ |
| F3 | 2226-20201103 | SY, SO, FP | SY, SO, FP | SY, SO, PO++ | SY, SO, FP | SY, SO, FP | SY, SO, FP | SY, SO, FP | SY, SO, PO++ |
| F4 | 2226-20201104 | SY, SO, FP | SY, SO, FP | SY, SO, PO++ | SY, SO, FP | SY, SO, PO++ | SY, SO, PO++ | SY, SO, PO+ | SY, SO, PO+ |
| F5 | 2226-20201105 | SY, SO, FP | SY, SO, FP | SY, SO, PO++ | SY, SO, FP | SY, SO, FP | SY, SO, FP | SY, SO, PO+ | SY, SO, FP |
| F6 | 2226-20201106 | SY, SO, FP | SY, SO, FP | SY, SO, PO++ | SY, SO, FP | SY, SO, FP | SY, SO, FP | SY, SO, FP | SY, SO, PO++ |
| F7 | 2226-20201107 | SY, SO, FP | SY, SO, FP | SY, SO, PO++ | SY, SO, FP | SY, SO, FP | SY, SO, FP | SY, SO, FP | SY, SO, PO++ |
| F8 | 2226-20201108 | SY, SO, FP | SY, SO, FP | SY, SO, PO++ | SY, SO, FP | SY, SO, FP | SY, SO, FP | SY, SO, FP | SY, SO, PO++ |
| F9 | 2226-20201109 | SY, SO, FP | SY, SO, FP | SY, SO, PO+ | SY, SO, FP | SY, SO, FP | SY, SO, FP | SY, SO, FP | SY, SO, PO+ |

| | | | | | | | | | |
|---|---|---|---|---|---|---|---|---|---|
| Notes: C (colorless)/SY (slightly yellow)/Y (yellow); CL (clear liquid)/SO (slightly opalescent liquid)/O (opalescent liquid); FP (free of visible particles)/PO (particles observed)/+ (a few visible particles)/++ (many visible particles). | | | | | | | | | |

### 2.3 Osmotic pressure, pH and protein concentration results of excipient and surfactant screening experiments

The osmotic pressure, pH and protein concentration results of the excipient and surfactant screening experiments were summarized in **Table 10.** The data showed that after subjected to repeated freezing and thawing, vibration and shaking, high-temperature (30°C ± 2°C) investigation, acceleration (25°C ± 2°C) and 2°C to 8°C investigations, the 9 formula samples did not show any obvious changes in terms of pH and protein concentrations.

**Table 10 pH and protein concentrations of excipient and surfactant screening experiments**

| **Formula no.** | **Sample no.** | **Osmotic pressure (mOsm/kg)** | **pH** | | | | | | | **Protein concentration (mg/ ml)** | | | | | | |
|---|---|---|---|---|---|---|---|---|---|---|---|---|---|---|---|---|
| | | | **T0** | **FT-5C** | **30-1W** | **30-2W** | **25-2W** | **25-1M** | **05-1M** | **T0** | **FT-5C** | **30-1W** | **30-2W** | **25-2W** | **25-1M** | **05-1M** |
| F1 | 2226-20201101 | 328 | 7.7 | 7.7 | 7.7 | 7.7 | 7.7 | 7.6 | 7.7 | 30.2 | 30.1 | 30.4 | 30.4 | 30.2 | 30.2 | 30.1 |
| F2 | 2226-20201102 | 329 | 7.7 | 7.7 | 7.7 | 7.6 | 7.7 | 7.7 | 7.7 | 30.1 | 30.2 | 30.3 | 30.4 | 30.3 | 30.3 | 30.2 |
| F3 | 2226-20201103 | 338 | 7.7 | 7.7 | 7.7 | 7.7 | 7.7 | 7.7 | 7.7 | 30.3 | 30.2 | 30.1 | 30.4 | 30.4 | 30.2 | 30.2 |
| F4 | 2226-20201104 | 331 | 7.0 | 7.1 | 7.0 | 7.1 | 6.9 | 7.0 | 6.9 | 29.9 | 30.1 | 29.8 | 30.2 | 30.1 | 29.8 | 29.9 |
| F5 | 2226-20201105 | 361 | 7.5 | 7.5 | 7.5 | 7.5 | 7.5 | 7.5 | 7.5 | 29.7 | 30.0 | 29.8 | 30.3 | 30.0 | 30.0 | 29.8 |
| F6 | 2226-20201106 | 337 | 7.7 | 7.7 | 7.7 | 7.7 | 7.7 | 7.7 | 7.7 | 30.3 | 30.3 | 30.2 | 30.4 | 30.3 | 30.2 | 30.1 |
| F7 | 2226-20201107 | 342 | 7.8 | 7.7 | 7.7 | 7.6 | 7.7 | 7.7 | 7.7 | 30.1 | 30.0 | 30.0 | 30.1 | 30.2 | 30.1 | 30.0 |
| F8 | 2226-20201108 | 303 | 7.7 | 7.7 | 7.7 | 7.7 | 7.7 | 7.7 | 7.7 | 30.2 | 30.5 | 30.3 | 30.4 | 30.3 | 30.3 | 30.3 |
| F9 | 2226-20201109 | 321 | 7.8 | 7.8 | 7.8 | 7.8 | 7.8 | 7.7 | 7.8 | 15.0 | 15.1 | 15.0 | 15.1 | 14.9 | 15.0 | 15.0 |

### 2.4 Insoluble microparticle detection (MFI) results of excipient and surfactant screening experiments

The insoluble microparticle detection (MFI) results of the excipient and surfactant screening experiments were summarized in **Table 11.** The freezing and thawing sample data showed that the number of insoluble microparticles in the F1 sample, which was frozen and thawed, was greater than those of the rest samples; after the samples were placed at 30°C for 2 weeks, the data showed that the number of insoluble microparticles in the F1 sample was greater than that of the rest samples, and the F4 sample was not subjected to an MFI test due to the presence of many visible particles; and after the samples were placed at 25°C for 1 month, the data showed that the number of insoluble microparticles in the F1 and F4 samples was higher than that of the rest samples.

**Table 11 Insoluble microparticle detection (MFI) results of excipient and surfactant screening experiments**

| **Formula no.** | **Sample no.** | **Number of insoluble microparticles (AR** < **0.85, #/mL)** | | | | | | | | | | | | | | | |
|---|---|---|---|---|---|---|---|---|---|---|---|---|---|---|---|---|---|
| | | **T0 (#/mL)** | | | | **FT-5C (#/mL)** | | | | **30-2W (#/mL)** | | | | **25-1M (#/mL)** | | | |
| | | **≥ 2 µm** | **≥ 5 µm** | **≥ 10 µm** | **≥ 25 µm** | **≥ 2 µm** | **≥ 5 µm** | **≥ 10 µm** | **≥ 25 µm** | **≥ 2 µm** | **≥ 5 pm** | **≥ 10 µm** | **≥ 25 µm** | **≥ 2 µm** | **≥ 5 µm** | **≥10 µm** | **≥ 25 µm** |
| F1 | 2226-20201101 | 213 | 55 | 25 | 9 | 2690 | 1044 | 539 | 32 | 2821 | 982 | 364 | 87 | 3402 | 1778 | 852 | 92 |
| F2 | 2226-20201102 | 227 | 81 | 32 | 2 | 1111 | 158 | 35 | 5 | 883 | 125 | 22 | 2 | 661 | 231 | 99 | 23 |
| F3 | 2226-20201103 | 330 | 84 | 23 | 4 | 1352 | 292 | 99 | 17 | 839 | 77 | 23 | 4 | 716 | 210 | 81 | 15 |
| F4 | 2226-20201104 | 413 | 143 | 55 | 5 | 1262 | 115 | 25 | 4 | NT | NT | NT | NT | 3158 | 1347 | 597 | 153 |
| F5 | 2226-20201105 | 99 | 28 | 5 | 0 | 1244 | 115 | 23 | 4 | 1398 | 376 | 79 | 12 | 1158 | 282 | 74 | 5 |
| F6 | 2226-20201106 | 464 | 48 | 12 | 0 | 1137 | 64 | 15 | 2 | 1011 | 195 | 45 | 0 | 840 | 128 | 15 | 2 |
| F7 | 2226-20201107 | 143 | 43 | 12 | 4 | 1581 | 132 | 37 | 10 | 882 | 186 | 64 | 7 | 706 | 171 | 55 | 17 |
| F8 | 2226-20201108 | 174 | 55 | 17 | 5 | 1782 | 123 | 37 | 5 | 996 | 184 | 59 | 7 | 754 | 241 | 91 | 15 |
| F9 | 2226-20201109 | 82 | 19 | 7 | 2 | 1170 | 145 | 33 | 2 | 1676 | 245 | 56 | 9 | 487 | 79 | 30 | 4 |

### 2.5 Purity (SEC-HPLC) results of excipient and surfactant screening experiments

The SEC-HPLC test results were summarized in **Table 12.** After the samples were placed at 25°C for 1 month and at 30°C for 2 weeks, the data showed that the decrease ranges of the F4 and F5 samples were both greater than those of the rest samples, the decrease range of F9 was smaller than those of the rest samples, and the decrease ranges of the rest samples were similar. After the samples were placed at 2°C to 8°C for 1 month, the data showed that the main-peak purity of the F5 sample was decreased by 2.1%, and the main-peak purities of the rest formula samples had no significant change. After the samples were freezed and thawed for 5 cycles, the data showed that the main-peak purity of the F8 sample was decreased by 10.3%, and the main-peak purities of the rest samples had no significant change.

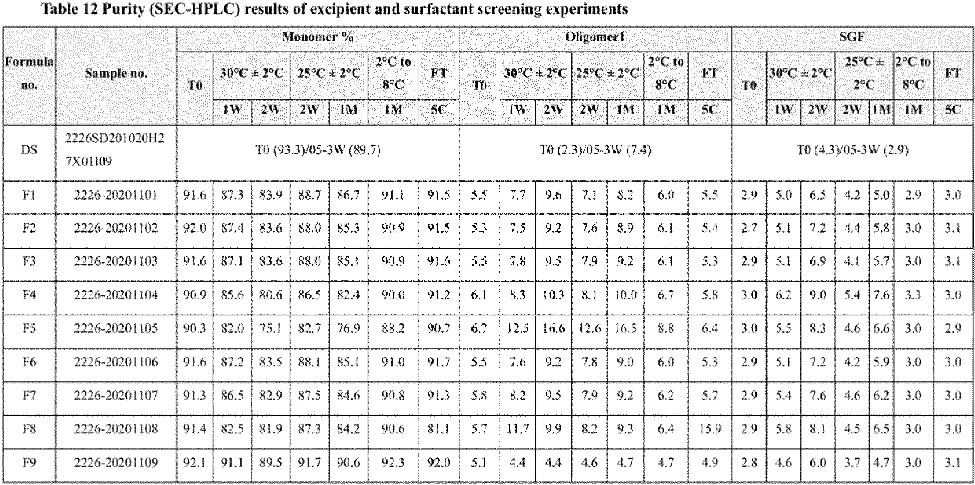

### 2.6 Purity (SDS-PAGE) results of excipient and surfactant screening experiments

The SDS-PAGE data were summarized in Table 13. The reducing SDS-PAGE data showed that the purities of different samples showed no significant difference. The non-reducing SDS-PAGE data showed that the purity of the F9 sample was superior to that of the rest samples, and the purity data of the rest samples were similar.

**Table 13 SDS-PAGE results of excipient and surfactant screening experiments**

| **Formula no.** | **Formula** | **SDS-PAGE-R (%) (H** + **L)** | | | | | | | **SDS-PAGE-NR (%)** | | | | | | |
|---|---|---|---|---|---|---|---|---|---|---|---|---|---|---|---|
| | | **T0** | **30°C ± 2°C** | | **25°C ± 2°C** | | **2°C to 8°C** | **FT** | **T0** | **30'C ± 2°C** | | 25°C **± 2°C** | | **2°C to 8°C** | **FT** |
| | | | **1W** | **2W** | **2W** | **1M** | **1M** | **5C** | | **1W** | **2W** | **2W** | **1M** | **1M** | **5C** |
| DS | 2226SD201020H27X01109 | 05-3W (99. 1) | | | | | | | 05-3W (91.6) | | | | | | |
| F1 | 2226-20201101 | 99.5 | 99.5 | 100.0 | 100.0 | 100.0 | 100.0 | 99.1 | 90.6 | 90.2 | 87.1 | 86.0 | 100.0 | 100.0 | 91.6 |
| F2 | 2226-20201102 | 99.4 | 99.5 | 100.0 | 100.0 | 100.0 | 100.0 | 99.1 | 91.3 | 92.0 | 88.5 | 86.7 | 87.9 | 92.3 | 91.5 |
| F3 | 2226-20201103 | 99.5 | 99.4 | 100.0 | 100.0 | 100.0 | 100.0 | 99.5 | 91.3 | 90.0 | 85.7 | 85.9 | 86.2 | 100.0 | 92.2 |
| F4 | 2226-20201104 | 99.3 | 99.2 | 100.0 | 100.0 | 100.0 | 100.0 | 99.2 | 91.5 | 89.0 | 86.2 | 87.3 | 100.0 | 100.0 | 91.3 |
| F5 | 2226-20201105 | 99.2 | 99.1 | 100.0 | 100.0 | 100.0 | 100.0 | 99.3 | 91.3 | 89.4 | 84.0 | 83.9 | 100.0 | 100.0 | 91.6 |
| F6 | 2226-20201106 | 99.5 | 99.2 | 100.0 | 100.0 | 100.0 | 100.0 | 99.5 | 92.1 | 90.0 | 87.9 | 86.8 | 100.0 | 100.0 | 92.1 |
| F7 | 2226--20201107 | 99.6 | 99.1 | 100.0 | 100.0 | 100,0 | 100.0 | 99.4 | 91.9 | 90.9 | 87.7 | 86.5 | 86.2 | 100.0 | 91.5 |
| F8 | 2226-20201108 | 99.6 | 99.1 | 100.0 | 100.0 | 100.0 | 100.0 | 99.4 | 91.4 | 92.2 | 94.5 | 90.3 | 100.0 | 100.0 | 92.2 |
| F9 | 2226-20201109 | 99.6 | 99.2 | 100.0 | 100.0 | 100.0 | 100.0 | 99.6 | 92.9 | 91.7 | 97.8 | 93.0 | 100.0 | 93.6 | 93.3 |

### 2.7 Summary of excipient and surfactant screening experiments

The appearance and insoluble microparticle determination results showed that polysorbate 80 was more conducive to avoiding the generation of microparticles in comparison with polysorbate 20; and the stabilizing effect of a glycine buffer system on the protein was weaker than that of a histidine buffer system.

SEC data showed that the protective effect of mannose on the IM001 was weaker than those of sucrose and trehalose. DSC and DLS results showed that the IM001 molecule was prone to chain scission and polymerization under low temperature conditions (40°C to 50°C). The data for F2 and F3 samples were similar under each investigation condition, indicating that sucrose and trehalose had similar stabilizing effects on the protein; the main-peak purities of the F4 and F5 samples under each investigation condition were slightly lower than those of the rest samples, indicating that the histidine buffer system at pH 8.0 was superior to the glycine and Tris buffer systems; the data for F2 and F6 samples were similar under each investigation condition, indicating that F6 in which I mM methionine was added and F2 in which methionine was not added showed no significant difference; and the data for F2 and F7 samples were similar under each investigation condition, indicating that the stability of F7, which comprises a combination of 4% mannitol and 2% sucrose was similar to that of F2, which comprises 250 mM sucrose. The main-peak purity of the F9 sample was higher than that of the F2 under each investigation condition, indicating that the aqueous formulation with 15 mg/mL proteins was more conducive to the stability of the IM001 in comparison with the aqueous formulation with 30 mg/mL proteins.

The reducing SDS-PAGE data showed that the purity data of different samples showed no significant difference. The non-reducing SDS-PAGE data showed that the purity of the F9 sample was superior to that of the rest samples, and the purity data of the rest samples were similar. F2 and F9 had the same composition except for different concentrations, indicating that the aqueous formulation with 15 mg/mL proteins was more conducive to the stability of the IM001 in comparison with the aqueous formulation with 30 mg/mL proteins.

### Example 3

The study purpose of this example was to screen the formula of a high-concentration lyophilized formulation.

Based on the formula screening results of the aqueous formulation, the concentration of the IM001 was increased to 30 mg/mL, and the purity was decreased obviously with the prolonged time of storage at 25°C or higher temperature. Therefore, the formula of a lyophilized formulation was designed. See Table 14 for the scheme. The IM001 stock solution was exchanged into a 25 mM histidine buffer system (pH 8.0); different excipient mother solutions and surfactant mother solutions were added respectively according to the experimental scheme; and the protein content was adjusted to 30 mg/mL by adding different kinds of sugar mother solutions and surfactant mother solutions, respectively. The 5 formulas prepared were filtered respectively with a 0.22-µm PVDF membrane in a biological safety cabinet, and then 5 mL of the solution was taken into cleaned and sterilized 20R penicillin bottles with a pipette; and the bottles were plugged, capped and labeled timely. Then, lyophilizing was performed, and stability investigation was started under different conditions. See Table 15 for the specific scheme.

**Table 14 Formula list of lyophilized formulation screening**

| **Formula no.** | **Composition and description of formula** |
|---|---|
| F2 | 30 mg/mL 25 mM His pH 8.0, 250 mM Sucrose, 0.02% PS80 (sample pH 7.6) |
| F7 | 30 mg/mL 25 mM His pH 8.0, 4% mannitol, 2% Sucrose, 0.02% PS80 (sample pH 7.6) |
| F15 | 30 mg/mL 25 mM His pH 8.0, 250 mM Trehalose, 0.02% PS80 (sample pH 7.6) |
| F16 | 30 mg/mL 25 mM His pH 8.0, 250 mM Sucrose, 0.04% PS80 (sample pH 7.6) |
| F17 | 30 mg/mL 25 mM His pH 8.0, 250 mM Sucrose, 0.02% PS80, 0.2% Poloxamer 188 (sample pH 7.6) |

**Table 15 Experimental scheme for stability of lyophilized products**

| **Formula no.** | **Type of stability** | **T0** | **Acceleration** | **High temperature** | | |
|---|---|---|---|---|---|---|
| | **Preservation condition** | | 25°C ± 2°C | 40°C | | |
| | **Sampling point** | | 3M | 2W | 1M | 2M |
| Lyo F2, F7, F15-F17 | Test items | X, Y, Z | X, Y | X | X, Y | X, Y |

| | | | | | | |
|---|---|---|---|---|---|---|
| wherein X = Appearance, osmotic pressure, pH, protein concentration, SEC-HPLC; Y = MFI; Z = Moisture content, Tc, Tg' | | | | | | |

### 3.1 Appearance observation of lyophilized products

The appearances of the lyophilized products were shown in FIG. 4, and all formulas were white, loose blocks after lyophilizing, wherein F7 (comprising 4% mannitol and 2% sucrose) was looser than other lyophilized products.

### 3.2 Moisture content determination of lyophilized products

The moisture content determination results of the lyophilized products were shown in Table 16. The moisture contents of different formulas were all less than 3%, which meets the moisture content standards of lyophilized products, and the specific moisture content was 1.1%-1.6%. The moisture content of F7 was 1.63% (slightly high).

**Table 16 Moisture content determination of lyophilized products**

| **Formula no.** | **Sample no.** | **Moisture content** |
|---|---|---|
| F2 | 2226-20210509 | 1.39% |
| F7 | 2226-20210510 | 1.63% |
| F15 | 2226-20210511 | 1.12% |
| F16 | 2226-20210512 | 1.17% |
| F17 | 2226-20210513 | 1.17% |

### 3.3 Glass-transition temperature (Tg') and lyophilizing collapse temperature (Tc) determination of frozen solutions of lyophilized formulas

See Table 17 for the glass-transition temperature (Tg') and lyophilizing collapse temperature (Tc) determination results of frozen solutions of the lyophilized formulas. The Tg' of formulas F2, F16 and F17 comprising sucrose was about -27°C; the Tg' of formula F7 comprising trehalose was -25.9°C (slightly high); and the Tg' of formula F7 comprising 4% mannitol and 2% sucrose was -33.2°C (the lowest). In addition, the Tc of the two formulas, F2 and F15, was determined as -23.8°C and -26.7°C respectively.

**Table 17 Glass-transition temperature (Tg') and lyophilizing collapse temperature (Tc) determination results of lyophilized solutions**

| **Formula no.** | **Sample no.** | **Tg'** | **Tc** |
|---|---|---|---|
| F2 | 2226-20210509 | -27.2°C | -23.8°C |
| F7 | 2226-20210510 | -33.2°C | NA |
| F15 | 2226-20210511 | -25.9°C | -26.7°C |
| F16 | 2226-20210512 | -27.7°C | NA |
| F17 | 2226-20210513 | -27.9°C | NA |

### 3.4 Appearance tests for reconstituted solutions of lyophilized products

Based on the mass change differences of the lyophilized products before and after lyophilizing, the volume of water added for reconstituting was obtained. After the lyophilized products were reconstituted by adding water, the appearances of the reconstituted solutions were observed. The results were shown in Table 18. For all samples, including T0, acceleration conditions (25°C) and high temperature conditions (40°C) caused the reconstituted solution being slightly yellow, slightly opalescent and free of visible particles.

**Table 18 Appearance determination results of reconstituted solutions of lyophilized products**

| **Formula no.** | **Sample no.** | **Appearance** | | | | |
|---|---|---|---|---|---|---|
| | | **T0** | **25C-3M** | **40C-2W** | **40C-1M** | **40C-2M** |
| F2 | 2226-20210509 | SY, SO, FP | SY, SO, FP | SY, SO, FP | SY, SO, FP | SY, SO, FP |
| F7 | 2226-20210510 | SY, SO, FP | SY, SO, FP | SY, SO, FP | SY, SO, FP | SY, SO, FP |
| F15 | 2226-20210511 | SY, SO, FP | SY, SO, FP | SY, SO, FP | SY, SO, FP | SY, SO, FP |
| F16 | 2226-20210512 | SY, SO, FP | SY, SO, FP | SY, SO, FP | SY, SO, FP | SY, SO, FP |
| F17 | 2226-20210513 | SY, SO, FP | SY, SO, FP | SY, SO, FP | SY, SO, FP | SY, SO, FP |

| | | | | | | |
|---|---|---|---|---|---|---|
| Notes: C (colorless)/SY (slightly yellow)/Y (yellow); CL (clear liquid)/SO (slightly opalescent liquid)/O (opalescent liquid); FP (free of visible particles)/PO (a few visible particles) | | | | | | |

### 3.5 pH, protein concentration and osmotic pressure detection of reconstituted solutions of lyophilized products

The pH, protein concentration and osmotic pressure determination results of the reconstituted solutions of the lyophilized products with different formulas were summarized in Table 19. The data showed that after 5 formula samples were placed under acceleration conditions (25°C) and high temperature conditions (40°C), the pH, protein concentrations and osmotic pressure showed no obvious changes.

**Table 19 pH, protein concentration and osmotic pressure determination results of reconstituted solutions of lyophilized products**

| **Formula no.** | t **Sample no.** | **pH** | | | | | **Protein concentration (mg/ml)** | | | | | **Osmotic pressure (mOsm/kg)** | | | | |
|---|---|---|---|---|---|---|---|---|---|---|---|---|---|---|---|---|
| | | **T0** | **25C-3M** | **40C-2W** | **40C-1M** | **40C-2M** | **T0** | **25C-3M** | **40C-2W** | **40C-1M** | **40C-2M** | **T0** | **25C-3M** | **40C-2W** | **40C-1M** | **40C-2M** |
| F2 | 2226-20210509 | 7.6 | 7.7 | 7.6 | 7.6 | 7.6 | 32.5 | 31.3 | 31.1 | 31.4 | 31.4 | 322 | 311 | 305 | 314 | 315 |
| F7 | 2226-20210510 | 7.6 | 7.7 | 7.6 | 7.6 | 7.6 | 30.3 | 31.5 | 30.1 | 29.8 | 30.2 | 332 | 358 | 336 | 333 | 339 |
| F15 | 2226-20210511 | 7.6 | 7.7 | 7.7 | 7.6 | 7.6 | 30.7 | 30.1 | 29.5 | 29.9 | 29.6 | 334 | 334 | 322 | 325 | 326 |
| F16 | 2226-20210512 | 7.6 | 7.7 | 7.6 | 7.6 | 7.6 | 31.1 | 31.0 | 30.9 | 31.3 | 30.9 | 310 | 322 | 314 | 324 | 312 |
| F17 | 2226-20210513 | 7.6 | 7.7 | 7.6 | 7.6 | 7.6 | 31.2 | 31.0 | 31.0 | 30.9 | 31.5 | 311 | 319 | 313 | 312 | 322 |

### 3.6 Insoluble microparticle detection (MFI) of reconstituted solutions of lyophilized products

The insoluble microparticle detection (MFI) results of the reconstituted solutions of the lyophilized products were summarized in Table 20. The number of insoluble microparticles in different formulas had no obvious differences, and in comparison with the T0 sample, the number of insoluble microparticles in the samples placed at 25°C for 1 month or 40°C for 2 months was not increased obviously after reconstituting.

**Table 20 Insoluble microparticle detection (MFI) results of reconstituted solutions of lyophilized products**

| **Formula no.** | **Sample no.** | **MFI [≥ 2 µm, 10 µm, 25 µm] #/ml** | | | | |
|---|---|---|---|---|---|---|
| | | **T0** | **25C-3M** | **40C-2W** | **40C-1M** | **40C-2M** |
| F2 | 2226-20210509 | 8068/457/91^{∗1} | 266/6/0 | NA | 746/55/12 | 1144/15/2 |
| F7 | 2226-20210510 | 905/35/0 | 1109/15/5 | | 530/30/4 | 3378/243/28 |
| F15 | 2226-20210511 | 891/81/2 | 13769/275/17^{∗2} | | 798/92/12 | 338/27/2 |
| F16 | 2226-20210512 | 939/197/38 | 335/10/2 | | 664/46/9 | 729/22/2 |
| F17 | 2226-20210513 | 957/213/27 | 763/12/2 | | 551/38/7 | 834/28/5 |

| | | | | | | |
|---|---|---|---|---|---|---|
| ^{*1,2} The two samples may be contaminated. As can be seen from the data in the Table, the insoluble microparticles in the 25C-3M, 40C-1M and 40C-2M samples of F2 were obviously fewer than those in the T0 sample, and with regard to F15, it can also be found that the number of insoluble microparticles in the 40C-1M and 40C-2M samples was obviously lower than that in the 25C-3M sample. | | | | | | |

### 3.7 Purity (SEC-HPLC) detection of reconstituted solutions of lyophilized products

The SEC-HPLC test results were summarized in Table 21. After all the formulas were placed under acceleration conditions (25°C) and high temperature conditions (40°C) for 2 weeks to 3 months, except that the SEC purity of F7 was lightly decreased, the SEC purities of the rest samples were generally unchanged, indicating that the stability of the lyophilized product was significantly improved in comparison with the liquid formulation. The stability of F7 (comprising 4% mannitol and 2% sucrose) was worse than that of the rest formulas, which may be related to the crystallization of mannitol after annealing and the loss of protective effects of sugar on the protein. The rest formulas had generally unchanged SEC main peak contents and all had good stability.

**Table 21 Purity (SEC-HPLC) test results of reconstituted solutions of lyophilized products**

| **Formula no.** | **Sample no.** | **SEC (Monomer/HMW/LMW, %)** | | | | |
|---|---|---|---|---|---|---|
| | | **T0** | **25C-3M** | **40C-2W** | **40C-1M** | **40C-2M** |
| F2 | 2226-20210509 | 95.8/4.1/0.1 | 95.8/4.1/0.1 | 95.6/4.3/0.1 | 95.2/4.8/0.1 | 95.7/4.3/0.1 |
| F7 | 2226-20210510 | 95.5/4.4/0.1 | 95.0/4.9/0.1 | 94.7/5.3/0.1 | 93.8/5.9/0.3 | 94.1/5.9/0.0 |
| F15 | 2226-20210511 | 96.0/3.9/0.1 | 95.9/4.0/0.1 | 95.7/4.3/0.1 | 95.2/4.7/0.1 | 95.6/4.3/0.1 |
| F16 | 2226-20210512 | 95.8/4.1/0.1 | 95.8/4.1/0.1 | 95.6/4.3/0.1 | 95.1/4.8/0.1 | 95.7/4.3/0.1 |
| F17 | 2226-20210513 | 95.8/4.1/0.1 | 95.8/4.1/0.1 | 95.6/4.4/0.1 | 95.2/4.7/0.1 | 95.7/4.3/0.1 |

### 3.8 Summary of lyophilized formulation screening experiments

The 5 lyophilized products investigated all had good appearances, and were white, loose blocks. The moisture content determination results were 1.1% to 1.6%, all less than 3%, so the moisture contents were qualified. The reconstituted solutions were all slightly yellow, slightly opalescent and free of visible particles after the products were placed under acceleration conditions (25°C) and high temperature conditions (40°C) for 2 weeks to 3 months. The Tg' of F7 (comprising 4% mannitol and 2% sucrose) was -33.2°C, and the Tg' of the rest formulas was about -26°C to -27°C. MFI results of different formulas showed no obvious differences. The SEC results showed that after investigation, all the formulas except for F7 had good stability after lyophilizing and no obvious decrease in their main peaks after being placed under acceleration conditions (25°C) for 3 months and high temperature conditions (40°C) for 2 months, and F7 (comprising 4% mannitol and 2% sucrose) showed a greater decrease in SEC main peak and poor stability in comparison with other formulas.

### Conclusions

The buffer system screening results show that the His buffer salt system at pH 8.0 (formula: pH 7.6) has the best stability.

The excipient and surfactant screening results show that the stability in the histidine buffer system at pH 8.0 is stronger than that in the glycine and Tris buffer systems; the protective effects of sucrose and trehalose are similar and both superior to that of mannitol; the system with a protein concentration of 15 mg/mL has a more superior stability than the system with a protein concentration of 30 mg/mL; and the aqueous formulation with F9 (15 mg/mL protein, 25 mM His, 250 mM Sucrose, and 0.02% PS80, pH 7.6) has the best stability.

The lyophilized formulation screening experiment results show that the lyophilized products always have an obviously improved stability in comparison with the solution formula, wherein F7 (30 mg/mL protein, 25 mM His pH 8.0, 4% Mannitol, 2% Sucrose and 0.02% PS80) shows slightly poorer performance, whereas the rest formulas all show excellent stability.

Based on the above-mentioned experimental results, F15 (30 mg/mL protein, 25 mM His, 250 mM Trehalose, and 0.02% PS80, pH 7.6) is selected as the formula of the lyophilized formulation of the IM001.

The contents mentioned above are merely preferable embodiments of the present invention, and are not intended to limit the present invention formally or materially. It should be pointed out that, for those skilled in the art, several improvements and supplements can also be made without departing from the method of the present invention, and such improvements and supplements shall also be considered to be within the scope of protection of the present invention. Those skilled in the art, without departing from the spirit and scope of the present invention, can use the technical contents disclosed above to make some equivalent variations such as alterations, modifications, evolutions, which are all equivalent embodiments of the present invention. Moreover, any equivalent variations such as alterations, modifications and evolutions made to the above-mentioned embodiments according to essential techniques of the present invention still fall within the scope of the technical solution of the present invention.

## Claims

1. An aqueous formulation, comprising a fusion protein, a 20-30 mM histidine salt, 220-280 mM trehalose and 0.01(w/v)%-0.03(w/v)% polysorbate 80, with a pH of 7.0-8.2, wherein the fusion protein comprises two monomers having an amino acid sequence as set forth in SEQ ID NO: 1, and the two monomers are connected by a plurality of disulfide bonds.

2. The aqueous formulation according to claim 1, wherein the aqueous formulation has a pH of 7.4-7.8.

3. The aqueous formulation according to claim 2, wherein the aqueous formulation has a pH of 7.6.

4. The aqueous formulation according to claim 1, wherein the aqueous formulation comprises a 24-26 mM histidine salt.

5. The aqueous formulation according to claim 4, wherein the aqueous formulation comprises a 25 mM histidine salt.

6. The aqueous formulation according to claim 1, wherein the aqueous formulation comprises 240-260 mM trehalose.

7. The aqueous formulation according to claim 6, wherein the aqueous formulation comprises 250 mM trehalose.

8. The aqueous formulation according to claim 1, wherein the aqueous formulation comprises 0.015(w/v)%-0.025(w/v)% polysorbate 80.

9. The aqueous formulation according to claim 8, wherein the aqueous formulation comprises 0.02(w/v)% polysorbate 80.

10. The aqueous formulation according to claim 1, further comprising a tonicity agent, a surfactant, an antioxidant, a preservative or a mixture thereof.

11. The aqueous formulation according to claim 1, wherein each of the fusion protein molecules comprises no more than six galactose-α-1,3-galactose moieties.

12. The aqueous formulation according to claim 11, wherein each of the fusion protein molecules comprises no more than three galactose-α-1,3-galactose moieties.

13. The aqueous formulation according to claim 11, wherein the fusion protein comprises glycans, wherein on average at least 52% of the glycans comprise one or more sialic acid residues.

14. The aqueous formulation according to claim 1, comprising the fusion protein at a concentration of at least 10 mg/mL.

15. The aqueous formulation according to claim 14, comprising the fusion protein at a concentration of at least 25 mg/mL.

16. The aqueous formulation according to claim 1, comprising the fusion protein at a concentration of at least 25 mg/mL, a 24-26 mM histidine salt, 240-260 mM trehalose and 0.015(w/v)%-0.025(w/v)% polysorbate 80, with a pH of 7.4-7.8.

17. The aqueous formulation according to claim 16, comprising the fusion protein at a concentration of 30 mg/mL, a 25 mM histidine salt, 250 mM trehalose and 0.02(w/v)% polysorbate 80, with a pH of 7.6.

18. The aqueous formulation according to any one of claims 1-17, comprising no additional amino acid salts at a concentration higher than 10 mM other than the histidine salt, or preferably comprising no additional amino acid salts.

19. The aqueous formulation according to claim 18, comprising no additional sugars at a concentration higher than 10 mM other than the trehalose, or preferably comprising no additional sugars other than the trehalose.

20. The aqueous formulation according to claim 19, which consists of the fusion protein at a concentration of 30 mg/mL, a 25 mM histidine salt, 250 mM trehalose and 0.02(w/v)% polysorbate 80, with a pH of 7.6.

21. The aqueous formulation according to any one of claims 1-20, for use in the treatment of an inflammatory disease or an IL-6-mediated condition in a human.

22. The aqueous formulation for use according to claim 21, wherein the inflammatory disease or IL-6-mediated condition is an inflammatory bowel disease, preferably wherein the treatment induces amelioration of the inflammatory bowel disease.

23. The aqueous formulation for use according to claim 21, wherein the inflammatory bowel disease is Crohn's disease or ulcerative colitis, preferably wherein the treatment maintains amelioration of the inflammatory bowel disease.

24. The aqueous formulation for use according to claim 21, wherein the inflammatory disease or IL-6-mediated condition is rheumatoid arthritis, psoriasis, uveitis or atherosclerosis.

25. The aqueous formulation for use according to claim 21, wherein the inflammatory disease or IL-6-mediated condition is colitis unrelated to inflammatory bowel disease, preferably wherein the colitis is radiation colitis, diverticular colitis, ischemic colitis, infectious colitis, celiac disease, autoimmune colitis or colitis caused by an allergy affecting the colon.

26. A dry formulation, which can be obtained by lyophilizing the aqueous formulation according to any one of claims 1-20.

27. A dry formulation, which can generate the aqueous formulation according to any one of claims 1-20 by adding water.
